# EUROPÄISCHE PATENTSCHRIFT

(11) **EP 0 456 092 B1**
(45) Veröffentlichungstag und Bekanntmachung des Hinweises auf die Patenterteilung: **06.09.1995**
(21) Anmeldenummer: 91107002.7
(22) Anmeldetag: 30.04.1991
(51) Int. Cl.: A61B 5/0235

(54) **Dämpfungsglied für Druckmessanordnungen**
Damping device for pressure measuring apparatus
Dispositif amortisseur pour appareil de mesure de pression

(30) Priorität: 07.05.1990 DE 4014591
(43) Veröffentlichungstag der Anmeldung: 13.11.1991
(73) Patentinhaber: Peter von Berg, Extrakorporale Systeme -Medizintechnik GmbH, 85614 Kirchseeon (DE)
(72) Erfinder: von Berg, Peter, W-8011 Neukeferloh (DE)
(74) Vertreter: von Bülow, Tam, Dr.

(56) Entgegenhaltungen:
- EP-A- 0 097 223
- EP-A- 0 328 105
- US-A- 4 779 625

## Beschreibung

Die Erfindung betrifft ein Dämpfungsglied für Druckmeßanordnungen gemäß dem Oberbegriff des Patentanspruches 1.

Zur kontinuierlichen Überwachung des arteriellen Blutdruckes wird häufig eine Druckmeßanordnung eingesetzt, die im wesentlichen aus einem in eine periphere Arterie eingeführten Katheter, einer flüssigkeitsgefüllten Übertragungsleitung und einem Drucksensor besteht, der die am Ende der Übertragungsleitung bestehenden Drucksignale in ein proportionales elektrisches Signal umwandelt, das dann in einem Sichtgerät angezeigt oder in sonstiger Weise ausgewertet wird. Bei der Übertragung sich dynamisch ändernder Drücke in geschlossenen Flüssigkeitssystemen tritt das Problem auf, daß durch Fehlanpassung des Drucksensors an die Flüssigkeitssäule Reflektionen auftreten, die zur Verfälschung des Meßergebnisses führen. Beispielsweise wird eine Druckwelle, die von Patienten zum Drucksensor läuft, von letzterem nicht vollständig absorbiert sondern teilweise reflektiert und fließt somit mit verringerter Amplitude in Richtung zum Patienten zurück, wo sie wiederum aufgrund einer Fehlanpassung erneut reflektiert wird. Durch diese mehrfache flektierten Druckwellen entstehen Überlagerungen, deren Resonanzfrequenz bei ungünstiger Dimensionierung nahe einer Oberwelle niederer Ordnung der Grundschwingung oder gar auf der Grundschwingung selbst liegt, was signifikante Verfälschungen des Meßsignales bewirken kann, die auch mit elektronischen Mitteln bei der Auswertung nicht eliminiert werden können.

Zur Abschwächung solcher verfälschender parasitärer Schwingungen ist es bekannt (US 4 431 009, US 4 335 729), in der Nähe des Druchsensors Dämpfungsglieder parallel zur Übertragungsleitung vorzusehen, die zumindest einen Teil der hochfrequenten Komponenten des Drucksignals absorbieren, so daß die Amplitude der reflektierten bzw. parasitären Schwingungen abgeschwächt wird.

Das Dämpfungsglied der US 4 431 009 ist als einstellbares Nadelventil ausgebildet, das damit einen veränderbaren Strömungswiderstand darstellt. Es ist als separate Baugruppe zwischen dem Druckmeßwandler und einem Drei-Wege-Ventil angeordnet. Dieses Ventil verbindet die vom Patienten zum Druckmeßfühler laufende Übertragungsleitung mit einer Infusionseinrichtung, da in den meisten Fällen bei der kontinuierlichen Blutdruckmessung die Übertragunngsleitung mit einer Infusionslösung gespült wird. Schließlich befindet sich in der Übertragungsleitung auch noch ein Absperrventil, um den Drucksensor von dem Meßsystem abzutrennen, was unter anderem zur Justierung des Sensors auf atmosphären Druck erforderlich ist.

Das Dämpfungsglied der US 4 335 729 ist ebenfalls als Nadelventil ausgebildet, das an einen Abzweig der Übertragungsleitung angeschlossen ist und eine vollständig von starren Wänden umgebene, dichte Kammer aufweist, in der sich ein Luftpolster befindet, das Schwingungen dämpft. Das einstellbare Nadelventil reguliert die Öffnungsweite der Verbindung zwischen der Übertragungsleitung und der Kammer.

Diese bekannten Dämpfungsglieder erfüllen zwar ihren Zweck, sie sind jedoch relativ aufwendig, was gerade bei einem Einmal-Artikel von Bedeutung ist. Auch wird durch diese zusätzliche, einzustellende Komponente das gesamte Meßsystem unübersichtlich und kann durch seine Bauform bedingt nur in bestimmter Entfernung vom Druckmeßwandler angeordnet werden, wodurch auch die Wirkung noch nicht optimal ist.

Die DE-PS 24 05 584 beschäftigt sich bei einem System zum impulsweisen Ausstoßen von Tröpfchen mit dem Problem von Reflektionen und schlägt vor, zur Unterdrückung reflektierter Druckwellen eine akustische Impedanzanpassung durch eine elastische Leitung zu realisieren.

Die DE-PS 29 41 118 zeigt einen Flüssigkeits-Feder-Dämpfer mit zwei topfförmigen Kammern, die über eine Scherfeder "schwimmend" gegeneinander gelagert sind. Beide Kammern stehen über eine Drosselstelle in Strömungsverbindung. Im Inneren der inneren Kammer ist ein elastischer Balg angeordnet, der mit einem einstellbaren Gasdruck beaufschlagt ist und bei entsprechendem Gasdruck die Drosselstelle absperrt. Erst wenn der Druck in der Hauptkammer größer ist als der in dem Balg, öffnet die Drosselstelle. Hierdurch erhält man ein Dämpfungsglied mit nicht-linearer und über den Druck im Inneren des Balges einstellbaren Charakteristik.

Aufgabe der Erfindung ist es, das Dämpfungsglied der eingangs genannten Art dahingehend zu verbessern, daß es kompakter aufgebaut ist.

Diese Aufgabe wird durch die Merkmale des Patentanspruches 1 gelöst. Vorteilhafte Ausgestaltungen und Weiterbildungen der Erfindung sind den Unteransprüchen zu entnehmen.

Kurz zusammengefaßt ist bei der Erfindung das Dämpfungsglied im Ventileinsatz eines Ventiles integriert. Der Durchflußweg des Ventileinsatzes steht über eine Kapillarbohrung mit einer Kammer im Ventileinsatz in Verbindung, die durch eine gummi-elastische Membran abgeschlossen ist. Vorzugsweise ist die Membran so abgestützt, daß sie sich primär nur nach einer Seite in Richtung einer Vergrößerung der Kammer verformen kann. An der von der Kammer wegweisenden Außenseite der Membran ist eine zweite Kammer vorhanden, die über eine Düse oder Öffnung mit dem Umgebungsdruck bzw. Atmosphäre in Verbindung steht. Damit sich die Membran primär nur in der einen Richtung verformen kann, ist der Durchmesser der zweiten Kammer größer als der der ersten Kammer.

Nach einer Variante der Erfindung kann die Düse bzw. Öffnung der zweiten Kammer auch fortgelassen werden, so daß die zweite Kammer dicht zur Umgebung abgeschlossen ist und damit eine konstantes Luftpolster zur Schwingungsdämpfung beinhaltet.

Nach einer weiteren Variante der Erfindung ist vorgesehen, die Bewegungsmöglichkeit der Membran zu begrenzen oder ganz zu verhindern, indem ein Stößel in die zweite Kammer eingeführt wird, der in einer Grenzlage mit seiner Stirnseite die Membran berührt und damit in ihrer Bewegung blockiert. In Zwischenstellungen des Stößels wird einerseits die maximale Auslenkamplitude der Membran begrenzt und andererseits wird das Volumen der zweiten Kammer variiert.

Vorzugsweise ist das Dämpfungsglied in den Ventileinsatz eines Drei-Wege-Ventiles integriert. Nach einer Variante der Erfindung kann es aber auch in andere Ventilarten integriert sein, beispielsweise auch in ein einfaches Absperrventil.

Im folgenden wird die Erfindung anhand von Ausführungsbeispielen im Zusammenhang mit der Zeichnung ausführlicher erläutert. Es zeigt:
- Fig. 1: einen Querschnitt des Dämpfungsgliedes im Ventileinsatz eines Drei-Wege-Ventiles nach einem ersten Ausführungsbeispiel der Erfindung;
- Fig. 2: einen Querschnitt eines Ventileinsatzes mit Dämpfungsglied nach einem zweiten Ausführungsbeispiel der Erfindung;
- Fig. 3: eine Seitenansicht eines Drei-Wege-Ventiles mit integriertem Dämpfungsglied und
- Fig. 4: eine Draufsicht des Ventiles der Fig. 3.

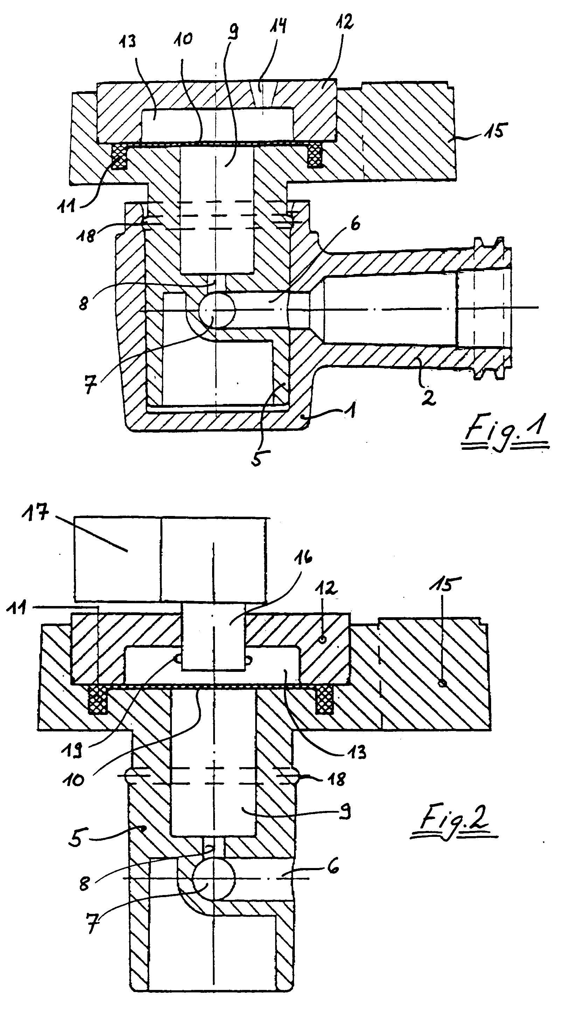

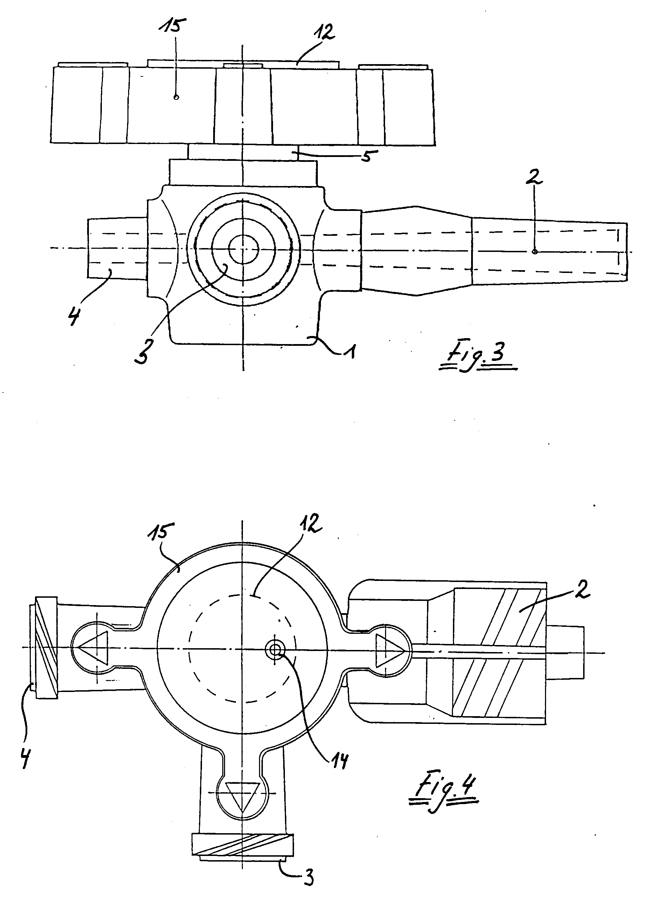

Das Drei-Wege-Ventil der Fig. 1 besitzt ein Gehäuse 1 mit drei Anschlußstutzen 2, 3, 4 (vgl. Fig. 3 und 4), in den ein Ventileinsatz 5 (Ventilküken) eingesetzt ist. Der Ventileinsatz hat die üblichen Durchlässe 6 und 7, die je nach Drehstellung des Ventileinsatzes die Strömungsverbindung zwischen den verschiedenen Anschlußstutzen 2, 3 bzw. 4 herstellen. Insoweit handelt es sich um ein herkömmliches Drei-Wege-Ventil.

In den Ventileinsatz 5 ist nun ein Dämpfungsglied integriert. Hierzu stehen die Durchlässe 6 und 7 des Ventileinsatzes 5 über eine Kapillarbohrung 8 mit einer ersten Kammer 9 in Strömungsverbindung, wobei diese Kammer im Ventileinsatz integriert ist und hier zylindrische Gestalt hat. Die der Kapillarbohrung 8 abgewandte Stirnseite der ersten Kammer 9 ist durch eine gummi-elastische Membran 10 abgeschlossen. Die Ränder der Membran 10 sind in einer Ringnut 11 gehalten, die in einem sich verbreiternden Griff 15 des Ventileinsatzes angeordnet ist. Der Durchmesser der Membran 10 ist deutlich größer als der Durchmesser der zylindrischen Erstkammer 9, so daß beträchtliche Teile am Rande der Membran auf der Stirnseite der die erste Kammer 9 begrenzenden Wandung aufliegen. Damit wird erreicht, daß die Membran 10 sich in größerem Umfange in Richtung einer Vergrößerung der ersten Kammer 9 verformen kann als in umgekehrter Richtung, d.h. in Richtung einer Verkleinerung der Kammer 9.

Die Membran 10 wird weiterhin durch einen Deckel 12 mit U-förmigem Querschnitt gehalten, der in eine Ausnehmung des Griffes 15 eingesetzt ist und zusammen mit der Membran 10 eine zweite Kammer 13 bildet. Der Durchmesser dieser zweiten Kammer 13 ist größer als der Durchmesser der ersten Kammer 9.

Der Deckel 12 besitzt eine düsenartige Öffnung 14, durch welche die zweite Kammer 13 mit Umgebungsdruck bzw. Atmosphäre beaufschlagt ist. Die Öffnung 14 ist so klein gewählt, daß sie einen Strömungswiderstand für die bei Auslenkung der Membran zwischen der Kammer 13 und Atmosphäre ausgetauschte Luft darstellt.

Beim Ausführungsbeispiel der Fig. 2 ist das Dämpfungsglied schaltbar, indem ein Stößel 16 in Axialrichtung verschiebbar im Deckel 12 angeordnet ist, daß er in seiner einen Grenzstellung die Membran berührt und sie dadurch am Schwingen hindert. Der Durchmesser des Stößels entspricht in etwa dem Durchmesser der ersten Kammer 9, so daß die Stirnseite des Stößels den Bereich der Membran 10, der die erste Kammer 9 verschließt, vollständig abdeckt. Im dargestellten Ausführungsbeispiel ist dieser Stößel mittels eines Gewindes 19, das eine relativ große Steigung hat, im Deckel 12 verschraubt. Zur Betätigung des Stößels 16 ist ein Hebel 17 vorgesehen. Alternativ kann ein Dreh-Kipphebel mit Exzenter vorgesehen sein, der die Auslenkung der Membrane wahlweise verhindert. Da das Gewinde zwischen dem Stift 16 und dem Deckel 12 nicht absolut dicht ist, wird dadurch auch die Funktion der Öffnung 14 des Ausführungsbeispieles der Fig. 1 realisiert.

Die Fig. 3 und 4 zeigen Ansichten eines 3-Wegeventiles mit dem integrierten Dämpfungsglied.

Eine gute Dämpfungscharakteristik im gesamten Meßkreis zu erhalten, kann das Ventil mit Dämpfungsglied unmittelbar an dem (nicht dargestellten) Meßwandler angeflanscht sein. Der in den Fig. 1 und 2 gezeigte Bund 18 am Ventileinsatz dient zur Arretierung des Ventileinsatzes im Gehäuse gegen Bewegungen in Axialrichtung.

## Patentansprüche

1. Dämpfungsglied für Druckmeßanordnungen, insbesondere für die Drucküberwachung bei der Blutdruckmessung, mit einem Durchlaßpfad (6, 7) und einer mit diesem in Strömungsverbindung stehenden Kammer (9), dadurch gekennzeichnet, daß das Dämpfungsglied im Ventilkörper (5) eines Ventiles integriert ist und daß die Kammer (9) durch eine gummi-elastische Membran (10) abgeschlossen ist.

2. Dämpfungsglied nach Anspruch 1, dadurch gekennzeichnet, daß die der Kammer (9) abgewandte Seite der Membran (10) eine zweite Kammer (13) abdichtet, die über eine düsenartige Öffnung (14) mit Atmosphärendruck verbunden ist.

3. Dämpfungsglied nach Anspruch 1 oder 2, dadurch gekennzeichnet, daß die erste Kammer (9) über eine Kapillarbohrung (8) mit dem Durchlaßpfad (6, 7) des Ventilkörpers (5) verbunden ist.

4. Dämpfungsglied nach einem der Ansprüche 1 bis 3, dadurch gekennzeichnet, daß die Membran so an dem Ventilkörper (5) angebracht ist, daß sie sich im wesentlichen nur in einer die erste Kammer (9) vergrößernden Richtung verformen kann.

5. Dämpfungsglied nach Anspruch 4, dadurch gekennzeichnet, daß der Durchmesser der ersten Kammer (9) kleiner ist als der Durchmesser der zweiten Kammer (13).

6. Dämpfungsglied nach einem der Ansprüche 2 bis 5, dadurch gekennzeichnet, daß die zweite Kammer durch einen Deckel (12) mit U-förmigem Querschnitt gebildet ist, der in den Ventilkörper (5) eingesetzt ist und mit seinem vorspringenden Rand die Membran (10) fixiert.

7. Dämpfungsglied nach einem der Ansprüche 1 bis 6, dadurch gekennzeichnet, daß eine schaltbare Sperreinrichtung (16) vorgesehen ist, mit der Auslenkungen der Membran (10) blockierbar sind.

8. Dämpfungsglied nach Anspruch 7, dadurch gekennzeichnet, daß die Sperreinrichtung ein Stößel (16) ist, der mit einem Gewinde (19) in den Deckel (12) einschraubbar ist, wobei das Gewinde (19) gleichzeitig als düsenartige Öffnung dient.

9. Dämpfungsglied nach einem der Ansprüche 1 bis 8, dadurch gekennzeichnet, daß es im Ventilkörper eines Drei-Wege-Ventiles integriert ist.

10. Dämpfungsglied nach einem der Ansprüche 1 bis 8, dadurch gekennzeichnet, daß es im Ventilkörper eines Absperrventiles integriert ist.

## Claims

1. A damping member for pressure-measuring arrangements, in particular for pressure monitoring when measuring blood pressure, comprising a duct path (6, 7) and a chamber (9) in flow connection therewith, characterised in that the damping member is incorporated into the valve body (5) of a valve and in that the chamber (9) is closed by a rubber-elastic diaphragm (10).

2. A damping member according to claim 1, characterised in that the side of the diaphragm (10) facing away from the chamber (9) seals a second chamber (13) communicating with atmospheric pressure via a nozzle-type opening (14).

3. A damping member according to claim 1 or 2, characterised in that the first chamber (9) is connected to the duct path (6, 7) of the valve body (5) by a capillary bore (8).

4. A damping member according to any one of claims 1 to 3, characterised in that the diaphragm is mounted on the valve body (5) in such a manner that it can deform substantially only in a direction enlarging the first chamber (9).

5. A damping member according to claim 4, characterised in that the diameter of the first chamber (9) is smaller than the diameter of the second chamber (13).

6. A damping member according to any one of claims 2 to 5, characterised in that the second chamber is formed by a cover (12) of U-shaped cross-section inserted into the valve body (5) and fixing the diaphragm (10) with its projecting edge.

7. A damping member according to any one of claims 1 to 6, characterised in that a controllable blocking device (16) is provided, by means of which deflections of the diaphragm (10) can be blocked.

8. A damping member according to claim 7, characterised in that the blocking device is a rod (16) screwable into the cover (12) by means of a thread (19) simultaneously functioning as a nozzle-type opening.

9. A damping member according to any one of claims 1 to 8, characterised in that it is incorporated into the valve body of a three-way valve.

10. A damping member according to any one of claims 1 to 8, characterised in that it is incorporated into the valve body of a shut-off valve.

## Revendications

1. Dispositif amortisseur pour appareils de mesure de pression, notamment pour surveiller la pression lors de la prise de la tension artérielle, comprenant une voie de passage (6, 7) et une chambre (9) qui est en connexion d'écoulement avec cette dernière, caractérisé en ce que le dispositif amortisseur est intégré dans le corps de soupape (5) d'une soupape et en ce que la chambre (9) est fermée par une membrane (10) élastique.

2. Dispositif amortisseur selon la revendication 1, caractérisé en ce que le côté de la membrane (10) qui est éloigné de la chambre (9) étanchéifie une deuxième chambre (13) qui est reliée à la pression atmosphérique par l'intermédiaire d'une ouverture (14) semblable à une buse.

3. Dispositif amortisseur selon l'une des revendications 1 ou 2, caractérisé en ce que la première chambre (9) communique avec la voie de passage (6, 7) du corps de soupape (5) par l'intermédiaire d'un alésage capillaire (8).

4. Dispositif amortisseur selon l'une des revendications 1 à 3, caractérisé en ce que la membrane est fixée sur le corps de soupape (5) de manière à ne pouvoir sensiblement se déformer que dans une direction agrandissant la première chambre (9).

5. Dispositif amortisseur selon la revendication 4, caractérisé en ce que le diamètre de la première chambre (9) est inférieur au diamètre de la deuxième chambre (13).

6. Dispositif amortisseur selon l'une des revendications 2 à 5, caractérisé en ce que la deuxième chambre est formée par un couvercle (12) ayant une section en forme de U, qui est inséré dans le corps de soupape (5) et fixe la membrane (10) grâce à son bord en saillie.

7. Dispositif amortisseur selon l'une des revendications 1 à 6, caractérisé en ce que l'on prévoit un dispositif de blocage (16) commutable au moyen duquel les excursions de la membrane (10) peuvent être bloquées.

8. Dispositif amortisseur selon la revendication 7, caractérisé en ce que le dispositif de blocage est un poussoir (16) pouvant être vissé dans le couvercle (12) grâce à un filetage (19), le filetage (19) servant en même temps d'ouverture semblable à une buse.

9. Dispositif amortisseur selon l'une des revendications 1 à 8, caractérisé en ce qu'il est intégré dans le corps de soupape d'une soupape à trois voies.

10. Dispositif amortisseur selon l'une des revendications 1 à 8, caractérisé en ce qu'il est intégré dans le corps de soupape d'une soupape d'arrêt.
